# EUROPEAN PATENT SPECIFICATION

(11) **EP 1 556 013 B1**
(45) Date of publication and mention of the grant of the patent: **13.05.2009**
(21) Application number: 03769384.3
(22) Date of filing: 14.10.2003
(51) Int. Cl.: A61K 9/20, A61K 31/41, A61P 39/04

(54) **DEFERASIROX DISPERSIBLE TABLETS**
DEFERASIROX DISPERGIERBARE TABLETTE
COMPRIMES SOLUBLES DE DEFERASIROX

(30) Priority: 15.10.2002 GB 0223978
(43) Date of publication of application: 27.07.2005
(73) Proprietor: NOVARTIS AG, 4056 Basel (CH); Novartis Pharma GmbH, 1230 Wien (AT)
(72) Inventor: DEFFEZ, Karine, F-69007 Lyon (FR); CASSIERE, Jean-Pierre, F-92500 Rueil Malmaison (FR)
(74) Representative: Roth, Peter Richard
(86) International application number: PCT/EP2003/011351
(87) International publication number: WO 2004/035026

(56) References cited:
- EP-A- 1 070 496
- WO-A-00/57886
- WO-A-97/49395

## Description

The present invention relates to dispersible tablets, e.g. pharmaceutical dispersible tablets, comprising 4-[3,5-bis(2-hydroxyphenyl)-[1,2,4]triazol-1-yl]benzoic acid (also called deferasirox) or a pharmaceutically acceptable salt thereof, and is hereinafter referred to as Compound I.

Compound I is an orally active iron chelator that is indicated in the treatment of iron overload in transfusion dependent anemias, in particular thalassemia major, thalassemia intermediate and in sickle cell disease to reduce iron-related morbidity and mortality. Compound I can also be used in the treatment of hemochromatosis.

Clinical thalassemia (major and intermedia) are hereditary disorders characterized by defective production of hemoglobin, which leads to decreased production and increased destruction of red blood cells.

Sickle cell disease is caused by a mutation in the hemoglobin-Beta gene leading to the production of abnormal hemoglobin S. Normal red blood cells die after 120 days and sickle cells (red blood cells with hemoglobin S) are destroyed more rapidly (10 to 20 days) causing anemia. This anemia is what gives the disease its commonly known name - sickle cell anemia.

Hemochromatosis, the most common form of iron overload disease, is an inherited disorder that causes the body to absorb and store too much iron. The extra iron builds up in organs and damages them. Without treatment, the disease can cause these organs to fail.

Patients with sickle cell disease or thalassemia, who receive significant numbers of blood transfusions and patients with hemochromatosis require therapy to remove iron from the body, called chelation therapy.

Compound I has the following formula:

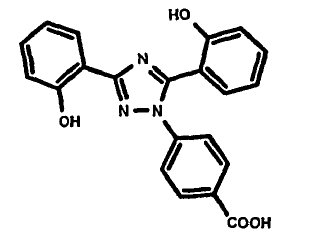

Compound I in the free acid form, salts thereof and its crystalline forms are disclosed in the International Patent Publication WO 97/49395 (published on December 31, 1997).

Typically, prescribed daily dosages of Compound I for the treatment of thalassemia are high, e.g. 5 to 40 mg/kg of body weight/day in adults or children. In children, the dosage is preferably 5 to 30 mg/kg of body weight/day. Due to the high dosage strength, the tablet dimensions do not permit the formulation of a conventional tablet. Thus, there is a need for an oral dosage form that is convenient to administer to adults and to children and that provides a pharmacologically active daily dosage amount of Compound I.

Present inventors have now surprisingly found that the formulation of Compound I in form of a dispersible tablet allows an oral dosage form with a high drug loading and which is convenient to administer to, for example children and elderly, and stable.

By "dispersible tablet" is meant a tablet which disperses in aqueous phase, e.g. in water, before administration.

Accordingly, the present invention provides a dispersible tablet with high drug loading comprising Compound I as active ingredient, the active ingredient being present in an amount of from about 5% to 40%, e.g. at least about 10, 15, 20 or 25 %, preferably more than 25% in weight based on the total weight of the dispersible tablet. In particular, the amount of Compound I may vary from 25 to 40%, e.g. 28 to 32% in weight based on the total weight of the dispersible tablet.

The present invention pertains to a dispersible tablet comprising an iron-chelating pharmacologically effective amount of Compound I or a pharmaceutically acceptable salt thereof present in an amount of from 5% to 40% weight by weight based on the total weight of the tablet and at least one disintegrant in a total amount of 10% to 35% in weight based on the total weight of the tablet.

In one aspect of the invention is provided a dispersible tablet comprising Compound I or a pharmaceutically acceptable salt thereof present in an amount of from 5% to 40% in weight based on the total weight of the tablet.

Compound I may be in the free acid form or pharmaceutically acceptable salts thereof, preferably in the free acid form. The active moiety corresponds to Compound I in the free acid form. Within the context of this disclosure, reference to Compound I is understood to include Compound I in its free acid form or a pharmaceutically acceptable salt thereof or any crystal forms thereof including hydrates or solvates, if not indicated otherwise and where appropriate and expedient.

The present invention also provides a dispersible tablet comprising:
(a) Compound I or a pharmaceutically acceptable salt thereof, and
(b) at least one pharmaceutically acceptable excipient suitable for the preparation of dispersible tablets wherein the amount of Compound I or a pharmaceutically acceptable salt thereof, calculated as the percentage of the content in weight of the active moiety based on the total weight of the dispersible tablet, is from about 5% to 40% t, e.g. at least about 10, 15, 20 or 25 %, preferably more than 25% in weight based on the total weight of the dispersible table. In particular, the amount of Compound I as active ingredient may vary from 25 to 40%, e.g. 28 to 32% in weight based on the total weight of the dispersible tablet.

In a preferred embodiment of the invention, the present invention provides a dispersible tablet wherein Compound I is in the free acid form (Compound I free acid form).

In a most preferred aspect of the invention, Compound I in the free acid form is in a crystalline form.

One or more pharmaceutically acceptable excipients may be present in the dispersible tablets, e.g. those conventionally used, e.g. (1.1) at least one filler, e.g., lactose, ethylcellulose, microcrystalline cellulose, (1.2) at least one disintegrant, e.g. cross-linked polyvinylpyrrolidinone, e.g. Crospovidone®, (1.3) at least one binder, e.g. polyvinylpyridone, hydroxypropylmethyl cellulose, (1.4) at least one surfactant, e.g. sodium laurylsulfate, (1.5) at least one glidant, e.g. colloidal silicon dioxide, (1.6), at least one lubricant, e.g. magnesium stearate.

Reference is made to the extensive literature on the subject for these and other pharmaceutically acceptable excipients and procedures mentioned herein, see in particular Handbook of Pharmaceutical Excipients, Third Edition, edited by Arthur H. Kibbe, American Pharmaceutical Association, Washington, USA and Pharmaceutical Press, London; and Lexikon der Hilfsstoffe für Pharmazie, Kosmetik and angrenzende Gebiete edited by H.P. Fiedler, 4th Edition, Edito Cantor, Aulendorf and earlier editions which are incorporated herein by reference.

Fillers (1.1) according to the invention are lactose especially lactose monohydrate, preferably lactose monohydrate (200mesh) and lactose spray dried, microcrystalline cellulose especially PH 102, PH 101.

Suitable disintegrants (1.2) according to the invention include but are not restricted to maize starch, CMC-Ca, CMC-Na, microcrystalline cellulose, cross-linked PVP, e.g. as known and commercially available under the trade names Crospovidone®, Polyplasdone®, available commercially from the ISP company, or Kollidon® XL, alginic acid, sodium alginate and guar gum. Preferably, cross-linked PVP, e.g. Crospovidone® is used.

Binders (1.3) include but are not restricted to starches, e.g. potato, wheat or corn starch, microcrystalline cellulose, e.g. products such as Avicel^{®}, Filtrak^{®}, Heweten^{®} or Pharmacel^{®}; hydroxypropyl cellulose, hydroxyethyl cellulose, hydroxypropylmethyl cellulose, e.g. hydroxypropylmethyl cellulose-Type 2910 USP, hypromellose, and polyvinylpyrrolidone, e.g. Povidone^{®} K30 from BASF. Preferably, polyvinylpyrrolidone is used, most preferably PVP K.30.

Appropriate surfactant (1.4) according to the invention may be used: sodium laurylsulfate, betain, quaternary ammonium salts, polysorbates, sorbitan erters and poloxamer. Preferably the surfactant is sodium laurylsulfate.

As glidants (1.5), one or more of the following may be used: silica; colloidal silica, e.g. colloidal silica anhydrous, e.g. Aerosil® 200, magnesium trisilicate, powdered cellulose, starch and talc. Preferably, colloidal silicon dioxide is used.

As lubricants (1.6) one or more of the following may be used Mg-, Al- or Ca-stearate, PEG 4000 - 8000, talc, sodium benzoate, glyceryl mono fatty acid, e.g. having a molecular weight of from 200 to 800 Daltons, e.g. glyceryl monostearate (e.g. Danisco, UK), glyceryl dibehenate (e.g. CompritolAT0888^{™}, Gattefossé France), glyceryl palmito-stearic ester (e.g. Precirol^{™}, Gattefossé France), polyoxyethylene glycol (PEG, BASF), hydrogenated cotton seed oil (Lubitrab, Edward Mendell Co Inc), castor seed oil (Cutina HR, Henkel). Preferably, magnesium stearate is used.

One or more of these pharmaceutically acceptable excipients may be selected and used having regard to the particular desired properties of the dispersible tablet by routine experimentation.

According to the present invention, the amount of filler (1.1) may vary within a range of from about 35 to 55%, in particular 40 to 50% in weight based on the total weight of the dispersible tablet.
The amount of disintegrant (1.2) may vary within a range of from 5 to 40%, e.g. 10 to 35% in weight based on the total weight of the dispersible tablet.
The amount of binder (1.3) may vary from 1 to 10 %, preferably from 1.5 to 5 % in weight based on the total weight of the dispersible tablet.
The amount of surfactant (1.4) may vary from 0.1 to 2%, preferably from 0.2 to 1%.
The amount of glidant (1.5) may vary within ranges of from 0.1 to 5%, in particular 0.1 to 2.5%, e.g. 0.1 to 0.5% in weight based on the total weight of the dispersible tablet.
The amount of lubricant (1.6) may be below 1% in weight based on the total weight of the dispersible tablet, preferably below 0.5%, most preferably below 0.4% and even most preferably the amount of lubricant is ranging between 0.01% and 0.4%. Very preferably the amount of lubricant is above 0.02% and below 0.4% in weight based on the total weight of the dispersible tablet.

It will be appreciated that any given excipient may serve more than one function e.g. as filler, disintegrant, binder, glidant, and/or lubricant.

In one aspect of the invention, a lubricant is present in less than 1% in weight based on the total weight of the dispersible tablet, preferably less than 0.4%.

The invention also pertains to a dispersible tablet wherein the lubricant is magnesium stearate.

In a preferred aspect of the invention, the dispersible tablet comprises the following pharmaceutically acceptable excipients: one or more fillers in a total amount of about 40% to 50% in weight based on the total weight of the dispersible tablet, one or more binders in a total amount of about 1.5% to 5% in weight based on the total weight of the dispersible tablet, one or more disintegrants in a total amount of about 10% to 35% in weight based on the total weight of the dispersible tablet, one or more glidants in a total amount of about 0.1% to 0.5% in weight based on the total weight of the dispersible tablet, and/or one or more lubricants in a total amount of about 0.01 % to 0.4 % in weight based on the total weight of the dispersible tablet.

In a preferred aspect of the invention, the dispersible tablet comprises the following pharmaceutically acceptable excipients: one or more fillers in a total amount of about 40% to 50% in weight based on the total weight of the dispersible tablet, one or more binders in a total amount of about 1.5% to 5% in weight based on the total weight of the dispersible tablet, one or more disintegrants in a total amount of about 10% to 35% in weight based on the total weight of the dispersible tablet, one or more surfactant in a total amount of about 0,2% to 1 % in weight based on the total weight of the dispersible tablet, one or more glidants in a total amount of about 0.1% to 0.5% in weight based on the total weight of the dispersible tablet, and/or one or more lubricants in a total amount of about 0.01 % to 0.4 % in weight based on the total weight of the dispersible tablet.

The absolute amounts of each pharmaceutically acceptable excipient and the amounts relative to other pharmaceutically acceptable excipients is similarly dependent on the desired properties of the dispersible tablet and may also be chosen by routine experimentation.

The present inventors have encountered difficulties in the production of dispersible tablets comprising Compound I which may be due to the low density of the active ingredient, to its electrostatic characteristics which may lead to a poor flowability and to its sticking tendency.

In accordance with the present invention, it has now unexpectedly been found that pharmaceutically acceptable oral solid dosage forms in the forms of dispersible tablets convenient for patient administration and dispersible in 5 minutes or less, preferably 3 minutes or less, may be obtained by preparation of tablets by compression methods. More specifically, the dispersible tablets of the invention may be prepared by granulation, preferably wet-granulation, followed by compression methods, preferably under spray lubrication.

In general, wet-granulation may be used to improve flowability and sticking tendency, however, wet-granulation process is not preferred when the pharmaceutical composition is to be a dispersible tablet. Indeed, wet-granulation increases the cohesion of the active ingredient particles and increases the disintegration time of the final tablet which is not in accordance with patient compliance or the European Pharmacopoeia which requests a disintegration time of 3 minutes or less for a dispersible tablet. Moreover, present inventors have encountered the problem that even upon wet-granulation the active ingredient remains sticky and is difficult to handle in a tabletting machine. Present inventors have now surprisingly found that stickiness may be resolved, e.g. by adding lubricant to the composition of the tablet without increasing the disintegration time, e.g. above acceptable values, e.g. above 5 minutes.

The dispersible tablets of the invention have a disintegration time, e.g. in aqueous media, e.g. in water, of 5 minutes or below 5 minutes. The dispersible tablets of the invention are, despite the high drug loading, dispersible, e.g. in aqueous media, e.g. in water, in less than 5 minutes, preferably less than 3 minutes, and, therefore, convenient to administer, e.g. to children or elderly. This leads to a better patient compliance.

In another embodiment, this invention provides a dispersible tablet comprising from 100 mg to 800 mg of Compound I as active ingredient, e.g. of from 100 mg to about 600 mg. Most preferably, dispersible tablets according to the invention are dispersible tablets containing 125 mg, 250 mg or 500 mg of Compound I as active ingredient.

Accordingly, the present invention provides dispersible tablets, e.g. dispersible tablets, containing an amount of Compound I, equal to 125 mg, 250 mg, or 500 mg of Compound I free acid form. Most preferably, the Compound I in the free acid form used for the dispersible tablet according to the invention is the crystalline form, especially the crystalline form the preparation of which is described in example 5 of WO 97/49395.

According to the invention, the process for the preparation of the dispersible tablets consists of granulating an inner phase, mixing (together) it with one or more pharmaceutically acceptable excipient(s) and compressing the obtained mixture under spray lubrication conditions.

The inner phase comprises Compound I. Preferably, the inner phase comprises Compound I and one or more pharmaceutically acceptable excipients. Preferably, the pharmaceutically acceptable excipients of the inner phase are one or more fillers, one or more disintegrants, one or more binders and one or more surfactants. Preferably the amount of one or more fillers in the inner phase is ranging from about 5 to 35% in weight based on the total weight of the dispersible tablet, more preferably 10 to 30% and most preferably 15 to 25%. The filler according to the invention is preferably lactose monohydrate. The disintegrant is preferably Crospovidone XL. The amount of disintegrant present in the inner phase is preferably ranging from 5 to 30%, more preferably 7 to 25% in weight based on the total weight of the dispersible tablet. The Compound I and one or more fillers and one or more disintegrants are mixed together with a wetting solution comprising one or more surfactants, water and one or more binders. The preferred binder is PVP K.30. The mixture is processed for granulation, e.g. using a wet high-shear granulator to form the wet-granulates. The wet-granulates may then be dried, e.g. using a fluid bed dryer, and calibrated, e.g. using an oscillating granulator. The outer phase consists of one or more pharmaceutically acceptable excipient(s) and is mixed with the inner phase using e.g. a free fall mixer. Preferably, one or more fillers and one or more glidants are added. Most preferably, cellulose microcrystalline and lactose are added as fillers. Even more preferably, microcrystalline cellulose is added in the range of 5 to 20% in weight based on the total weight of the dispersible tablet and lactose is added in the range of 5 to 20% in weight based on the total weight of the dispersible tablet. The outer phase according to the invention may also contain one or more glidants, most preferably colloidal silicon dioxide. In a preferred embodiment, the amount of glidant in the outer phase is ranging from about 0.1 to 5%, preferably 0.1 to 2.5%, most preferably 0.1 to 0.5% in weight based on the total weight of the tablet.

In one aspect of the invention one or more lubricants, instead of being incorporated into the mixture of the inner and outer phase, may be deposited on the punches of the tabletting machine before compression. According to the invention, one or more lubricants may be sprayed on the material contacting surfaces of pressing tools, e.g. punches and/or dies, of the tabletting machine before compression. Preferably, one or more lubricants are sprayed on the material contacting surfaces of pressing tools, e.g. punches and dies, of the tabletting machine before compression.

In one embodiment of the invention, the process for the preparation of a dispersible tablet comprises
(a) forming an inner phase comprising
   (i) mixing Compound I together with pharmaceutically acceptable pharmaceutically acceptable excipients,
   (ii) wet-granulating the mixture obtained in (i);
(b) forming an outer phase comprising
   (iii) adding further pharmaceutically acceptable excipients to the inner phase obtained in (ii) and mixing;
(c) forming the dispersible tablet by
   (iv) compressing the mixture obtained in step (iii) under spray lubrication condition.

In a further aspect the present invention provides a process comprising:
(i) mixing Compound I and pharmaceutically acceptable excipients, e.g. one or more fillers, e.g. lactose, and one or more disintegrants, e.g. Crospovidone XL, in a high shear mixer;
(ii) adding a solution of one or more surfactant and one or more binder, subjecting the mixture to wetting/kneading, e.g. in a high shear mixer, wet-granulating using, e.g. a rotating impeller, drying, e.g. in a fluidized bed dryer, then calibrating in an oscillating granulator, and;
(iii) adding pharmaceutically acceptable excipients, e.g. sieved excipients, such as one or more fillers, e.g. microcrystalline cellulose or lactose, one or more glidant, e.g. colloidal silicon dioxide, and mixing, e.g. in a free fall mixer;
(iv) tabletting the mixture obtained in step (iii) by compression, e.g. in a conventional tablet press, preferably a rotary machine and spraying the lubricant on the materials contacting surfaces of pressing tools.

Procedures which may be used may be conventional or known in the art or based on such procedures e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

By "inner phase" is meant the granulate phase (steps (i) and (ii)) including the active ingredient Compound I and one or more the pharmaceutically acceptable excipients.

By "outer phase" is meant one or more pharmaceutically acceptable excipients added to the inner phase (granulates) (step (iii).

By "total weight of the dispersible tablet" is meant the weight of a tablet being the inner and the outer phase.

The physical and chemical stability may be tested in conventional manner, e.g. the dispersible tablets may be tested as such by measurement of dissolution, friability, disintegration time, assay for Compound I degradation products, appearance and/or microscopy, e.g. after storage at room temperature, i.e. 25°C, and/or storage at 40°C.

The dispersible tablets may vary in shape and be, for example, round, oval, oblong, cylindrical or any other suitable shape. In one aspect, the dispersible tablets according to the invention contain small amount of magnesium stearate, e.g. about 0.01 % to 0.4 % in weight based on the total weight of the dispersible tablet, having regard to the amount of Compound I contained therein, thus allowing a disintegration time, which complies with the European Pharmacopoeia Specifications.

In a preferred embodiment of the invention dispersible tablets obtained by the compression method described above are round or oval. The edges of the dispersible tablets may be beveled or rounded. Most preferably, the dispersible tablets are round with bevelled edges. The dispersible tablets according to the invention may be scored, embossed or engraved.

The dispersible tablet according to the invention is preferably round, flat with bevelled edges. The 125 mg dispersible tablet has a diameter ranging between 10 and 20 mm, most preferably between 10 and 15 mm. The preferred diameter of the 125 mg dispersible tablet is 12 mm. Its thickness is ranging from 2.5 to 4.5 mm, preferably between 3.2 and 3.9 mm. The 250 mg dispersible tablet has a diameter ranging from 12 to 20 mm, preferably between 14 and 18 mm, the most preferred diameter is 15 mm. Its thickness is ranging from 3.5 to 5.5 mm, most preferably between 4 and 5 mm. The 500 mg dispersible tablet has a diameter ranging from 15 to 30 mm, preferably between 15 and 25 mm, the most preferred diameter is 20 mm. Its thickness is ranging from 4.5 to 6.5 mm, most preferably between 5 and 6 mm.

The dispersible tablets of the invention comprising about 125 mg of Compound I as active moiety may have a hardness of from about 50 to 120 N, preferably 60 to 100 N.
The dispersible tablets of the invention comprising about 250 mg of Compound I may have a hardness of 70 to 150 N, preferably 90 to 130 N. The dispersible tablets of the invention comprising about 500 mg of Compound I may have a hardness of 80 to 190 N, preferably 110 to 160 N.

Preferably, the disintegration time is not more than 5 minutes, most preferably the disintegration time is less than 3 minutes as measured using a disintegration time apparatus.

By "disintegration time" is meant the time that needs the dispersible tablet to disintegrate in water at room temperature in a disintegration time device.

The dispersible tablet of the present invention is dispersible in an aqueous phase, preferably water.

The dispersible tablets of the invention may be colored and/or marked so as to impart an individual appearance and to make them instantly recognizable. The use of dyes can serve to enhance the appearance as well as to identify the dispersible tablets. Dyes suitable for use in pharmacy typically include carotinoids, iron oxides or chlorophyll. The dispersible tablets of the invention may be marked using an imprint code.

The dispersible tablets of the invention are useful for the treatment of iron overload in transfusion dependent anemias, in particular thalassemia major, thalassemia intermediate and sickle cell disease and in the treatment of hemochromatosis.

The activity and characteristics of the dispersible tablets of the invention may be indicated in standard clinical trials and/or animal trials.

The dispersible tablets of the invention are stable both to the production process and during storage, e.g. for 2 years or even 3 years in conventional packaging, e.g. sealed aluminium blister packs or triplex blister packs. Less than about 5%, e.g. 2 or 3% or less of Compound I as active ingredient may degrade during this time as determined in conventional tests. For example, less than 1% of Compound I as active ingredient is degraded in one year in HDPE filled bottles.

Depending on age, individual condition, mode of administration, and the clinical picture in question, effective daily dosing, e.g. 350 to 2800 mg of Compound I, are administered to patients of 70 kg body weight.

The invention further relates also to a method of administering to a mammal, preferably a human subject, in need of such a treatment, Compound I in the form of a dispersible tablet. The invention relates especially to such method wherein a daily dose of 5 to 40 mg/kg of body weight of Compound I as active ingredient is administered to a patient. It will be understood that the specific dose level for any particular patient will depend upon a variety of factors including the age, the body weight, general health, drug combination with one or more active drugs, type and severity of the disease.

The invention further provides a medicament package comprising dispersible tablets according to the invention and printed instructions directing that one or more dispersible tablets of Compound I be administered orally.

The following non-limitative examples illustrate the invention.

### Example 1: Dispersible tablet Formulation (125 mg. 250 mg and 500 mg dispersible tablets) with a disintegration time above 3 minutes.

| | | | **Amount per dispersible tablet (mg)** | | |
|---|---|---|---|---|---|
| **Components** | | **%** | **125 mg** | **250 mg** | **500 mg** |
| Phase I | Compound I (free acid form) | 29.41 | 125.0 | 250.00 | 500.0 |
| | Lactose 200 Mesh (1.1) | 22.09 | 93.88 | 187.75 | 375.5 |
| | Crospovidone XL (1.2) | 10.00 | 42.50 | 85.00 | 170.00 |
| Phase II | PVP K.30 (1.3) | 3.00 | 12.75 | 25.50 | 51.00 |
| | Sodium laurylsulfate (1.4) | 0.50 | 2.13 | 4.25 | 8.50 |
| Phase III | Crospovidone XL (1.2) | 10.00 | 42.50 | 85.00 | 170.00 |
| | Microcrystalline cellulose (1.1) | 11.90 | 50.57 | 101.15 | 202.3 |
| | Lactose spray dried (1.1) | 11.90 | 50.57 | 101.15 | 202.3 |
| | Aerosil 200 (1.5) | 0.20 | 0.85 | 1.70 | 3.40 |
| Phase IV | Magnesium stearate (1.6) | 1.00 | 4.25 | 8.50 | 17.00 |
| **Tablet weight (mg)** | | 100.00 | 425.00 | 850.00 | 1700.00 |
| **Tablet diameter (mm)** | | - | 12 | 15 | 20 |

### Example 2: Dispersible tablet Formulation (125 mg, 250 mg and 500 mg dispersible tablets) with a disintegration time below 3 minutes.

| | | | **Amount per dispersible tablet (mg)** | | |
|---|---|---|---|---|---|
| **Components** | | **%** | **125 mg** | **250 mg** | **500 mg** |
| Phase I | Compound I (free acid form) | 29.4 | 125.0 | 250.0 | 500.0 |
| | Lactose 200 Mesh (1.1) | 17.1 | 72.6 | 145.2 | 290.4 |
| | Crospovidone XL (1.2) | 15.0 | 63.7 | 127.4 | 254.8 |
| Phase II | PVP K.30 (1.3) | 3.0 | 12.8 | 25.6 | 51.2 |
| | Sodium laurylsulfate (1.4) | 0.5 | 2.1 | 4.2 | 8.4 |
| Phase III | Crospovidone XL (1.2) | 5.0 | 21.3 | 42.6 | 85.2 |
| | Microcrystalline cellulose (1.1) | 14.9 | 63.3 | 126.6 | 253.2 |
| | Lactose spray dried (1.1) | 14.9 | 63.3 | 126.6 | 253.2 |
| | Aerosil 200 (1.5) | 0.2 | 0.9 | 1.8 | 3.6 |
| Phase IV | Magnesium stearate (1.6) | < 0.2* | | | |
| **Tablet weight (mg)** | | 100.0 | 425 | 850 | 1700 |
| **Tablet diameter (mm)** | | - | 12 | 15 | 20 |
| **Tablet thickness (mm)** | | - | 3.6+/-0.3 | 4.5+/-0.3 | 5.5+/-0.3 |

Dispersible tablets of Compound I free acid according to the invention are prepared by forming a inner phase by wet granulation of a mixture of Phase I and Phase II ingredients, Phase III ingredients formed the outer phase and the lubricant (Phase IV) is sprayed directly onto the punches of the tabletting machine. * 0.1% w/w of magnesium stearate is equivalent to 1000 ppm.

### Example 3: Properties of the 125 mg dispersible tablet of Example 2

| **Test** | **Release specifications** |
|---|---|
| Tablet shape | 12 mm diameter, round, flat, bevelled edge with engraving (IA/NVR) |
| Tablet appearance | Off white |
| Friability | Max. 1% (0 unit broken) |
| Crushing strength (mean) | Mean >= 70N |
| Dissolution rate | Q=75 % within 30 min |
| Disintegration time | All units <= 3.0 min |
| Average mass | 403.75- 446.25 mg |
| Mass uniformity | 18/20 within +/- 5% |
| | 20/20 within +/- 10% |
| Content uniformity | 10/10 units within 85.0% - 115.0 % / RSD <=6.0% |
| Mean content | 95.0 -105.0 % |
| Determination of degradation products | unknown degradation product: |
| | individual <=0.5% |
| | total <=2.0% |
| Fineness of dispersion | Smooth dispersion which passes through a sieve screen with a nominal mesh aperture of 710 µm |

### Example 4: Properties of the 250 mg dispersible tablet of Example 2

| **Test** | **release specifications** |
|---|---|
| Tablet shape | 15 mm diameter, round, flat, bevelled edge with engraving (IB/NVR) |
| Tablet appearance | Off white |
| Friability | Max. 1% (0 unit broken) |
| Crushing strength (mean) | Mean >=90N |
| Dissolution rate | Q =75 % within 30 min |
| Disintegration time | All units < 3 min |
| Average mass | 807.5 - 992.5 mg |
| Mean content | 95.0 -105.0 % |

### Example 5: Properties of the 500 mg dispersible tablet of Example 2

| **Test** | **Release specifications** |
|---|---|
| Tablet shape | 20 mm diameter, round, flat, bevelled edge with engraving (IC/NVR) |
| Tablet appearance | Off white |
| Friability | Max. 1% (0 unit broken) |
| Crushing strength (mean) | Mean >= 110N |
| Dissolution rate | Q =70 % within 30 min |
| Disintegration time | All units <= 3.0 min |
| Average mass | 1615 -1785 mg |
| Mass uniformity | 18/20 within +/- 5% |
| | 20/20 within +/-10% |
| Mean content | 95.0 -105.0 % |
| Content uniformity | 10/10 units within 85.0% - 115.0 % / RSD <=6.0% |
| Determination of degradation products | unknown degradation product: |
| | individual <=0.5% |
| | total <=2.0% |
| Fineness of dispersion | Smooth dispersion which passes through a sieve screen with a nominal mesh aperture of 710 µm |

### Example 6: Magnesium Stearate Assay

| | 125 mg tablet | | | 250 mg tablet | | 500 mg tablet | | |
|---|---|---|---|---|---|---|---|---|
| | L.P. | Pilot | FSP | P.T. | FSP | L.P. | Pilot | FSP |
| Min (%w/w) | 0.1 | 0.09 | 0.04 | 0.08 | 0.04 | 0.04 | 0.03 | 0.04 |
| Max (%w/w) | 0.24 | 0.36 | 0.14 | 0.16 | 0.08 | 0.10 | 0.12 | 0.06 |

| | | | | | | | | |
|---|---|---|---|---|---|---|---|---|
| L.P.: laboratory phase, Pilot: Pilot phase (x 2 of the size of the batch of the laboratory phase), FSP: (batches of production size), P.T.: preliminary trial, 0.1% w/w of magnesium stearate is equivalent to 1000 ppm. RSD: relative standard deviation | | | | | | | | |

### Example 7: Properties of the 250 mg dispersible tablet of Example 2

| **Test** | **Release specifications** |
|---|---|
| Tablet shape | 15 mm diameter, round, flat, bevelled edge with engraving (IB/NVR) |
| Tablet appearance | Off white |
| Friability | Max. 1% (0 unit broken) |
| Crushing strength (mean) | Mean >= 90N |
| Dissolution rate | Q =75 % within 30 min |
| Disintegration time | All units <= 3.0 min |
| Average mass | 807.5 - 892.5 mg |
| Mass uniformity | 18/20 within +/- 5% |
| | 20/20 within +/- 10% |
| Content uniformity | 10/10 units within 85.0% - 115.0 % / RSD<=6.0% |
| Mean content | 95.0 -105.0 % |
| Determination of degradation products | unknown degradation product: |
| | individual <=0.5% |
| | total <=2.0% |
| Fineness of dispersion | Smooth dispersion which passes through a sieve screen with a nominal mesh aperture of 710 µm |

## Claims

1. A dispersible tablet comprising Compound 1 of the formula 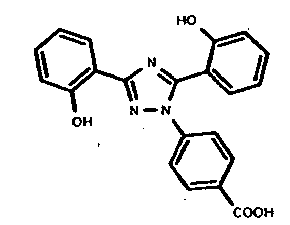 or a pharmaceutically acceptable salt thereof present in an amount of from 5% to 40% in weight based on the total weight of the tablet and (b) at least one disintegrant in a total amount of 10% to 35% in weight based on the total weight of the tablet.

2. The dispersible tablet according to claim 1 wherein the Compound I is present in an amount of 25 to 40% in weight based on the total weight of the tablet.

3. The dispersible tablet according to claim 2 wherein Compound I is present in an amount of 28 to 32% in weight based on the total weight of the tablet.

4. The dispersible tablet according to claim 1, 2 or 3 comprising an iron-chelating pharmacologically effective amount of Compound I or a pharmaceutically acceptable salt thereof.

5. The dispersible tablet according to any on of claims I to 4 wherein Compound I is in the free acid form.

6. The dispersible tablet according to any one of claims I to 5 wherein Compound I is in a crystalline form.

7. The dispersible tablet according to any one of claims 1 to 6 wherein a lubricant is present in less than 1% in weight based on the total weight of the tablet.

8. The dispersible tablet according to claim 7 wherein a lubricant is present in less than 0.4% in weight based on the total weight of the tablet.

9. The dispersible tablet according to anyone of claims 1 to 8 wherein the disintegration time of the tablet is of 3 minutes or less.

10. The dispersible tablet according to any one of claims 1 to 9 wherein the pharmaceutically acceptable excipients comprise:
(i) at least one filler in a total amount of 35 to 55 % in weight based on the total weight of the tablet,
(ii) at least one binder in a total amount of 1.5% to 5% in weight based on the total weight of the tablet,
(iii) at least one surfactant in a total amount of 0.2% to 1% in weight based on the total weight of the tablet,
(iv) at least one glidant in a total amount of 0.1% to 0.5% in weight based on the total weight of the tablet, and/or
(v) at least one lubricant in a total amount of less than 0.4% in weight based on the total weight of the tablet.

11. The dispersible tablet according to claim 10 wherein the amount of lubricant is in an amount of 0.01 % to 0.4% in weight based on the total weight of the tablet.

12. The dispersible tablet according to anyone of claims 1 to 10 containing Compound I in its free acid form in an amount of about 100 mg to 600 mg .

13. A process for the preparation of the dispersible tablet according to any one of the preceding claims, which process comprises
(i) mixing the Compound I or a pharmaceutically acceptable salt thereof and at least one pharmaceutically acceptable excipient;
(ii) wet-granulating the mixture obtained in (i);
(iii) mixing the granulates obtained in (ii) with at least one pharmaceutically acceptable excipient to form a mixture; and
(iv) spraying the lubricant on the materials contacting surfaces of pressing tools of the tabletting machine and compressing the mixture obtained in step (iii) to form a tablet.

14. The dispersible tablet according to any one of claims 1 to 12 or the process according to claim 13 wherein the lubricant is magnesium stearate.

## Patentansprüche

1. Dispergierbare Tablette, umfassend die Verbindung I der Formel 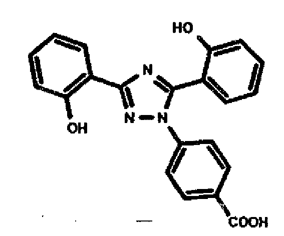 oder ein pharmazeutisch akzeptables Salz hiervon, in einer Menge von 5 Gew.-% bis 40 Gew.-%, bezogen auf das Gesamtgewicht der Tablette, und wenigstens ein Zerfallhilfsmittel in einer Menge von 10 Gew.-% bis 35 Gew.-%, bezogen auf das Gesamtgewicht der Tablette.

2. Dispergierbare Tablette nach Anspruch 1, worin die Verbindung I in einer Menge von 25 bis 40 Gew.-%, bezogen auf das Gesamtgewicht der Tablette, vorhanden ist.

3. Dispergierbare Tablette nach Anspruch 2, worin die Verbindung I in einer Menge von 28 bis 32 Gew.-%, bezogen auf das Gesamtgewicht der Tablette, vorhanden ist.

4. Dispergierbare Tablette nach Anspruch 1, 2 oder 3, umfassend eine pharmazeutisch wirksame Menge einer Verbindung der Formel I oder ein pharmazeutisch akzeptables Salz hiervon als Chelatbildner für Eisen.

5. Dispergierbare Tablette nach einem der Ansprüche 1 bis 4, worin die Verbindung I in Form der freien Säure vorliegt.

6. Dispergierbare Tablette nach einem der Ansprüche 1 bis 5, worin die Verbindung I in einer Kristallform vorliegt.

7. Dispergierbare Tablette nach einem der Ansprüche 1 bis 6, worin ein Schmiermittel in einer Menge von weniger als 1 Gew.-%, bezogen auf das Gesamtgewicht der Tablette, vorhanden ist.

8. Dispergierbare Tablette nach Anspruch 7, worin das Schmiermittel in einer Menge von weniger als 0,4 Gew.-%, bezogen auf das Gesamtgewicht der Tablette, vorhanden ist.

9. Dispergierbare Tablette nach einem der Ansprüche 1 bis 8, worin die Zerfallzeit der Tablette 3 min oder weniger beträgt.

10. Dispergierbare Tablette nach einem der Ansprüche 1 bis 9, worin die pharmazeutisch akzeptablen Hilfsstoffe der Tablette umfassen
(i) wenigstens einen Füllstoff in einer Gesamtmenge von 35 bis 55 Gew.-%, bezogen auf das Gesamtgewicht der Tablette,
(ii) wenigstens ein Bindemittel in einer Gesamtmenge von 1,5 Gew.-% bis 5 Gew.-%, bezogen auf das Gesamtgewicht der Tablette,
(iii) wenigstens ein oberflächenaktives Mittel in einer Gesamtmenge von 0,2 Gew.-% bis 1 Gew.-%, bezogen auf das Gesamtgewicht der Tablette,
(iv) wenigstens ein Gleitmittel in einer Gesamtmenge von 0,1 Gew.-% bis 0,5 Gew.-%, bezogen auf das Gesamtgewicht der Tablette, und/oder
(v) wenigstens ein Schmiermittel in einer Gesamtmenge von weniger als 0,4 Gew.-%, bezogen auf das Gesamtgewicht der Tablette.

11. Dispergierbare Tablette nach Anspruch 1, worin die Menge an Schmiermittel 0,01 Gew.-% bis 0,4 Gew.-%, bezogen auf das Gesamtgewicht der Tablette, beträgt.

12. Dispergierbare Tablette nach einem der Ansprüche 1 bis 10, welche die Verbindung I in Form der freien Säure in einer Menge von etwa 100 mg bis 600 mg enthält.

13. Verfahren zur Herstellung der dispergierbaren Tablette nach einem der vorhergehenden Ansprüche, umfassend eine
(i) Vermischung der Verbindung I oder eines pharmazeutisch akzeptablen Salzes hiervon und wenigstens eines pharmazeutisch akzeptablen Hilfsstoffs,
(ii) Nassgranulation des nach Stufe (i) erhaltenen Gemisches,
(iii) Vermischung der gemäß Stufe (ii) erhaltenen Granulate mit wenigstens einem pharmazeutisch akzeptablen Hilfsstoff unter Bildung eines Gemisches und
(iv) Aufsprühung des Schmiermittels auf die Materialien kontaktierenden Oberflächen von Presswerkzeugen der Tablettiermaschine und Kompression des in der Stufe (iii) erhaltenen Gemisches unter Bildung einer Tablette.

14. Dispergierbare Tablette nach einem der Ansprüche 1 bis 12 oder Verfahren nach Anspruch 13, worin das Schmiermittel Magnesiumstearat ist.

## Revendications

1. Comprimé dispersible comprenant un composé I de formule suivante : 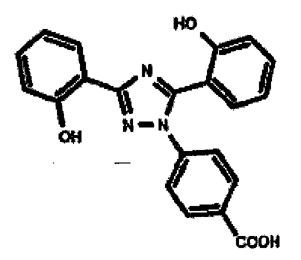 ou l'un de ses sels acceptable sur le plan pharmaceutique, en une quantité comprise entre 5 et 40 % en poids, sur la base du poids total du comprimé, et b) au moins un agent désintégrant en une quantité totale comprise entre 10 et 35 % en poids, sur la base du poids total du comprimé.

2. Comprimé dispersible selon la revendication 1, dans lequel le composé I est présent en une quantité comprise entre 25 et 40 % en poids, sur la base du poids total du comprimé.

3. Comprimé dispersible selon la revendication 2, dans lequel le composé I est présent en une quantité comprise entre 28 et 32 % en poids, sur la base du poids total du comprimé.

4. Comprimé dispersible selon la revendication 1, 2 ou 3, comprenant une quantité efficace sur le plan pharmacologique en terme de chélation du fer de composé I ou de l'un de ses sels acceptable sur le plan pharmaceutique.

5. Comprimé dispersible selon l'une quelconque des revendications 1 à 4, dans lequel le composé I se présente sous forme d'un acide libre.

6. Comprimé dispersible selon l'une quelconque des revendications 1 à 5, dans lequel le composé I se présente sous forme cristalline.

7. Comprimé dispersible selon l'une quelconque des revendications 1 à 6, dans lequel un lubrifiant est présent à raison de moins de 1 % en poids, sur la base du poids total du comprimé.

8. Comprimé dispersible selon la revendication 7, dans lequel un lubrifiant est présent à raison de moins de 0,4 % en poids, sur la base du poids total du comprimé.

9. Comprimé dispersible selon l'une quelconque des revendications 1 à 8, dans lequel le temps de désintégration du comprimé est de 3 minutes ou moins.

10. Comprimé dispersible selon l'une quelconque des revendications 1 à 9, dans lequel les excipients acceptables sur le plan pharmaceutique comprennent :
i) au moins une charge, en une quantité totale comprise entre 35 et 55 % en poids, sur la base du poids total du comprimé,
ii) au moins un liant, en une quantité totale comprise entre 1,5 et 5 % en poids, sur la base du poids total du comprimé,
iii) au moins un agent tensioactif, en une quantité totale comprise entre 0,2 et 1 % en poids, sur la base du poids total du comprimé,
iv) au moins un agent de glissement, en une quantité totale comprise entre 0,1 et 0,5 % en poids, sur la base du poids total du comprimé, et/ou v) au moins un lubrifiant, en une quantité totale inférieure à 0,4 % en poids, sur la base du poids total du comprimé.

11. Comprimé dispersible selon la revendication 10, dans lequel la quantité de lubrifiant est comprise entre 0,01 et 0,4 % en poids, sur la base du poids total du comprimé.

12. Comprimé dispersible selon l'une quelconque des revendications 1 à 10, comprenant le composé I sous forme d'un acide libre, en une quantité comprise entre environ 100 et 600 mg.

13. Procédé de préparation du comprimé dispersible selon l'une quelconque des revendications précédentes, comprenant :
i) le mélange du composé I ou de l'un de ses sels acceptable sur le plan pharmaceutique avec au moins un excipient acceptable sur le plan pharmaceutique ;
ii) la granulation par voie humide du mélange obtenu en i) ;
iii) le mélange des granulés obtenus en ii) avec au moins un excipient acceptable sur le plan pharmaceutique pour former un mélange ; et
iv) la pulvérisation du lubrifiant sur les surfaces des outils compresseurs de la machine à comprimer qui sont au contact du matériau, et la compression du mélange obtenu à l'étape iii) pour former un comprimé.

14. Comprimé dispersible selon l'une quelconque des revendications 1 à 12 ou procédé selon la revendication 13, dans lequel le lubrifiant est le stéarate de magnésium.
